# NEW EUROPEAN PATENT SPECIFICATION

(11) **EP 1 362 099 B2**
(45) Date of publication and mention of the opposition decision: **22.12.2021**
(45) Mention of the grant of the patent: 15.05.2013
(21) Application number: 02710764.8
(22) Date of filing: 06.02.2002
(51) Int. Cl.: C12N 9/99

(54) **METHOD OF PROVIDING POLYPEPTIDE PREPARATIONS WITH REDUCED ENZYMATIC SIDE ACTIVITIES**
VERFAHREN ZUR HERSTELLUNG VON POLYPEPTIDZUBEREITUNGEN MIT REDUZIERTER ENZYMATISCHER NEBENWIRKUNGEN
TECHNIQUE DE PREPARATIONS DE POLYPEPTIDE A ACTIVITES SECONDAIRES ENZYMATIQUES REDUITES

(30) Priority: 09.02.2001 US 779560
(43) Date of publication of application: 19.11.2003
(73) Proprietor: Chr. Hansen A/S, 2970 Hørsholm (DK)
(72) Inventor: HARBOE, Marianne, Kirsten, DK-2900 Lyngby (DK)
(74) Representative: Hoffmann Eitle
(86) International application number: PCT/DK2002/000083
(87) International publication number: WO 2002/064759

(56) References cited:
- WO-A-97/20921
- WO-A1-90/02799
- WO-A1-97/20921
- WO-A2-95/29999
- WO-A2-03/052092
- DD-A1- 280 331
- US-A- 2 683 682
- US-A- 2 683 682
- US-A- 3 950 221
- US-A- 3 950 221
- US-A- 4 100 024
- US-A- 4 324 805
- US-A- 5 021 338
- US-A- 5 151 358
- US-A- 5 215 908
- US-A- 5 679 543
- US-A- 6 103 508
- US-B2- 6 951 731
- Scopes RK. Protein Purification: Principles and Practice. 3rd Ed. 1994
- Tanaka T. and Yada R.Y.; Biochem. J. 1996. 315: 443-446
- Richter C. et al. Eur. J. Biochem. 1999, 261: 746-752
- Baker L.E., J. Biol. Chem. 1951, 193: 809-819
- Pedersen V.B. et al. Eur. J. Biochem. 1979, 94: 573-580
- Schlamowitz M. and Peterson L.U., J. Biol. Chem. 1959, 234: 3137-3145
- Zaldivar-Aguero J.M. et al. Braz. J. Chem. Eng. 1997, 14: 1-5
- Flanagan P.R. and Forstner G.G., Biochem. J. 1978, 173: 553-563
- Sopanen T. and Mikola J., Plant Physiol. 1975, 55: 809-814
- Verissimo P. et al. Eur. J. Biochem. 1996, 235: 762-768
- Morris B.J. and Lumbers E.R., Biochim. Biophys. Acta. 1972, 289: 385-391
- Vassar R. et al. Science. 1999, 286: 735-741
- Kimura H. et al. J. Biochem. 1975, 77: 909-912
- Toogood H.S. et al. Biochem. J. 1995, 307: 783-789
- Bustamante M. et al. J. Dairy Res. 2000, 67: 393-402
- Houen G. et al. Int. J. Biochem. Cell Biol. 1996, 28: 667-675
- Brown R.J. and Ernstrom C.A., Milk-Clotting Enzymes and Cheese Chemistry Part I - Milk-Clotting Enzymes, from Fundamentals of Dairy Chemistry, 3rd Edition (published 1988)
- A Guide to pH Measurement - the theory and practice of laboratory pH applications, Mettler Toledo (published 2007)
- Springer EK, pH Measurement Guide, Hamilton (published 2006)
- Bezkornovainy A. and Rafelson M.E., Concise Biochemistry (published 1996)
- White J.S. and White D.C., Source book of enzymes (published 1997)
- Anson M.L. and Mirsky A.E., The Equilibrium Between Active Native Trypsin and Inactive Denatured Trypsin. J Gen Physiol. 1934, 17: 393-398
- Excerpt from MEROPS Peptidase Database

## Description

### FIELD OF INVENTION

The present invention relates generally to processes of obtaining preparations of polypeptides having a low content of undesired enzymatic activities (side activities). In particular, the invention provides a simple and convenient process whereby the level of such side activities can be reduced significantly or substantially completely inactivated in crude and more or less purified polypeptide preparations such as preparations of aspartic proteases including chymosin species and microbial aspartic proteases.

### TECHNICAL BACKGROUND AND PRIOR ART

A large range of polypeptide products including enzymes and pharmaceutically active products are currently manufactured and made commercially available. Such products may be derived from a variety of sources. Thus, they can be derived from extracts of plant, animal or microbial cells naturally producing the products or they can be manufactured using appropriate recombinant microbial host organisms producing the desired product(s) either being accumulated intracellularly or excreted into the cultivation medium. In any case, the primary crude extract or medium resulting from the cultivation of the recombinant organisms normally contain, in addition to the desired product(s), a range of undesired enzymatic activities naturally produced by the source organisms or the recombinant host cells. In certain instances, an undesired enzymatic activity in a cultivation medium for a recombinant organism is derived from the fact that the desired product is produced as a fusion protein of the desired gene product and a fusion partner having, in relation to the final product, an undesired enzymatic side activity.

It is therefore a continuing concern for the industry how to provide polypeptide products that do not contain undesired enzymatic side activities or at least have a content of such activities at such a low level that they do not restrict the applicability of the polypeptide products for their intended purposes.

One illustrative example of such a problem is the that facing manufacturers of milk clotting enzymes, also referred to as rennets or coagulants. Milk clotting enzymes, which belong to the class of aspartic proteases, are widely used in the cheese manufacturing industry to provide a curd of the major milk proteins, the caseins. Commercially available milk clotting enzymes include native enzymes derived from microbial species or animal tissue sources such as calf stomachs, or such enzymes can be provided as gene products of recombinant cells expressing a heterologous milk clotting enzyme of animal or microbial origin.

The industrially most important milk clotting enzymes of animal origin are chymosin and pepsin. When produced in the animal gastric mucosal cells, chymosin and pepsin occur as enzymatically inactive pre-prochymosin and pre-pepsinogen, respectively. When chymosin is excreted, an N-terminal peptide fragment, the pre-fragment (signal peptide) is cleaved off to give prochymosin including a pro-fragment. Prochymosin is a substantially inactive form of the enzyme which, however, becomes activated under acidic conditions to the active chymosin by autocatalytic removal of the pro-fragment. This activation occurs *in vivo* in the gastric lumen under appropriate pH conditions or *in vitro* under acidic conditions.

Traditionally, milk clotting enzymes of bovine origin have been marketed in the form of extracts of stomach tissues. However, bovine chymosin is increasingly being manufactured industrially using recombinant DNA technology, e.g. using filamentous fungi such as *Aspergillus* species (see e.g. Ward, 1990), yeast strains, e.g. of *Klyuveromyces* species, or bacterial species, e.g. *E. coli,* as host organisms.

Milk clotting enzymes of microbial origin are currently in commercial use in the dairy industry. In the following, such enzymes are also referred to as microbial clotting enzymes, microbial rennets or microbial coagulants. Examples of such enzymes include aspartic proteases natively produced by the filamentous fungal species *Rhizomucor miehei* and *Rhizomucor pusillus* and protease naturally produced by the fungal species *Cryphonectria parasitica.* Enzymes having milk clotting activity are also produced naturally by other fungal species including *Rhizopus* species, *Physarum* species and *Penicillium* species, and *Bacillus* species.

Generally, currently applied processes for manufacturing of commercial rennet products include steps of recovering the active enzymes from crude extracts or microbial fermentation media, followed by one or more purification steps.

As one example, WO 90/15866 discloses a method for recovering and purifying chymosin from an aqueous solution which includes the addition of polyethylene glycol and an inorganic salt so as to form a two phase system whereby the chymosin is concentrated in the PEG phase and cell debris and other impurities in the salt phase, followed by recovering the chymosin-rich phase and isolating the chymosin herefrom chromatographically. Such a multi-step process, however, is associated with several problems: (i) the process is labour intensive and the use of salts and PEG adds to the production cost, (ii) the use of PEG requires strict measures to be taken to remove this substance and (iii) the use of large amounts of salt represents a significant pollution problem. Furthermore, and importantly, the enzyme preparation obtained by eluting it from the chromatographic column may have an undesirably high content of enzymatic side activities requiring a further separation step, e.g. chromatography, to remove these undesired activities

Attempts have been made to develop a simpler process for recovery of milk clotting enzymes from crude aqueous media. Thus, WO 95/29999 discloses the recovery of chymosin using a one-step chromatography process avoiding the use of PEG and salt.

However, such one-step processes frequently result in unsatisfactory yields of milk clotting enzyme, in particular when the conductivity of the crude preparation, such as a filtrate of a microbial fermentation medium, which is applied to the chromatographic column, is high. Another very significant problem associated with such processes is that also in such a process, the eluate from the column may have undesired enzymatic side activities and thus a further chromatography step is required to remove such activities.

WO 97/20921 relates to selective inactivation of enzyme activities. In example 4 it is disclosed that amylase can be inactivated by subjecting an amylase/ cellulase composition to a pH of 3.5. US 2683682 discloses that α-amylase is substantially inactivated leaving a relatively high proteinase activity when the pH is adjusted to within the limits 3.0 to 4.7.

The present invention is based on the surprising discovery that undesired enzymatic activities in polypeptide preparations can be reduced or eliminated by a very simple process step of subjecting the preparation to low pH for an appropriate period of time without any significant concurrent inactivation of the active polypeptide contained in the preparation.

This achievement has made it possible to develop an efficient, cost-effective and less polluting method of recovering and isolating active polypeptides in a one-step chromatographic process from aqueous media in the form of preparations not containing undesired levels of enzymatic side activities.

### SUMMARY OF THE INVENTION

Accordingly, in one aspect, the invention pertains to a method of providing a polypeptide preparation having a reduced content of undesired enzymatic side activities, the method comprising the steps of:
(i) providing a medium having a pH of 2.0 or higher which is derived from the cultivation of an organism that is selected from the group consisting of a bacterial species, a yeast species and a species of filamentous fungi and that comprises at least one desired polypeptide which has aspartic protease activity, wherein the aspartic protease is chymosin, and in addition hereto at least one undesired enzymatic side activity, and
(ii) subjecting said medium to a pH in the range of 1.0 to 1.8 for a period of time that is sufficient to at least partially inactivate the at least one enzymatic side activity.

In a further aspect there is provided a milk clotting composition comprising an aspartic protease provided by the method of the invention, said composition essentially not having undesired enzymatic side activities.

### DETAILED DISCLOSURE OF THE INVENTION

It is a major objective of the present invention to provide a method whereby a preparation containing a biologically active polypeptide having a reduced content of undesired enzymatic side activities can be obtained whilst at the same time substantially retaining the biological activity of the polypeptide.

In the present context, the term "preparation containing a biologically active polypeptide" refers to any preparation containing a desired polypeptide which is manufactured and sold for a particular intended purpose, the term "polypeptide" encompasses peptides of two or more amino acids, i.e. the term encompasses molecules that are also generally referred to as peptides, oligopeptides or proteins. The term "biologically active" is meant to include any detectable activity that can be detected in in vitro systems and in vivo in both prokaryotic and eukaryotic organisms including higher organisms such as animals and plants. Thus, the term refers to e.g. antimicrobial, pharmaceutical, immunological (antibodies or antigens) or enzymatic activity. Additionally, as used herein the term "preparation" includes preparations in any form that is suitable for application or administration of the preparations, such as dry, semi-dry and liquid forms including e.g. suspensions, solutions or emulsions.

It is, as it is mentioned above, a common observation that preparations containing one or more desired polypeptides including raw materials and intermediate products used in the manufacturing of final products containing the polypeptide has a content of undesired enzymatic activities, also referred to herein as side activities. In many instances, such side activities have a detrimental effect, which is exerted upon application of the desired polypeptide. As one typical example hereof, preparations of enzymes produced by extraction from the tissues of higher organisms or produced by cultivation of microorganisms, frequently contain minor amounts of undesired enzymatic activity. E.g. when milk clotting enzymes of both animal and microbial origin are produced using either organisms naturally producing such an enzyme or using recombinant host microorganisms having an inserted gene expressing the milk clotting enzyme, the producing strains produce a range of such side activities as described in the following. These side activities may restrict the field of application for the milk clotting enzyme and it is therefore common to set standards for content of side activities in such products which should be met.

The term "undesired enzymatic side activity" as used herein refers to any enzymatic activity, the presence of which in a polypeptide preparation is undesired for any reason such as detrimental or toxic effects occurring upon application or administration of the polypeptide preparation. As examples of such effects can be mentioned degradation of valuable components in a food product and immunologically adverse effects upon administration of a pharmaceutically active polypeptide. Undesired enzymatic activities encompassed by the present invention include as examples protease activity, starch degrading activitiy, peptidase activity, lipase activity, cellulase activity, lactase activity, hemicellulase activity, glucoamylase activity and phosphatase activity.

In the manufacturing of commercial polypeptide preparations the enzymatic side activity standards may be met by subjecting the products or the starting materials herefore to extensive purification processes which, however, in addition to the costs involved frequently implies loss of yield of the desired polypeptide. In accordance with the method of the invention, the level of undesired side activities is reduced or eliminated using a very simple approach, i.e. by subjecting a starting material, an intermediate material or the final product having a pH 2.0 or higher that comprises at least one desired polypeptide and in addition hereto at least one undesired enzymatic side activity to a treatment where the pH is adjusted to a pH in the range of 1.0 to 1.8 for a period of time that is sufficient to at least partially inactivate the at least one enzymatic side activity.

This acid treatment can be carried out using any suitable organic or inorganic acid, the use of which is compatible with the desired polypeptide and the applications herefore. A non-limiting range of such acids include lactic acid, acetic acid, citric acid, HCl, H₃PO₄, and H₂SO₄.

In preferred embodiments, this treatment at low pH results in that at least 75% of the activity of at least one desired polypeptide is retained after subjecting the medium having a pH of 2.0 or more to a pH in the range of 1.0 to 1.8. More preferably, at least 80% such as at least 85% and most preferably, at least 90%, e.g. at least 95% of the activity of the desired polypeptide is retained.

Preferably, the method of the invention is capable of destroying or inactivating at least 50% of the activity of at least one undesired enzymatic activity present in the polypeptide preparation. In certain instances a lower degree of inactivation may be fully acceptable if this lower degree of inactivation results in that the final product meets the standard specifications for the particular side activity/activities. More preferably, at least 60% of the side activity is inactivated by treatment, such as at least 70%, 80% or at least 90% of the activity of the undesired enzymatic side activity.
The media to be treated are media derived from cultivation of cells of a bacterial species such as a gram negative bacterial species including E. coli and a gram positive species including a Bacillus species, a yeast species and a species of filamentous fungi.

Thus, media which can be treated by the method of the invention include media derived from the cultivation of cells of a yeast species selected from Saccharomyces cerevisiae, a methylotrophic yeast species including Pichia pastoris and a Klyuveromyces species, and media from cultivation of species of filamentous fungi such as e.g. Aspergillus species, Cryphonectria species, Fusarium species, Rhizomucor species and Trichoderma species. The undesired enzymatic activities in such cultivation media are primarily enzymatic side activities that are produced naturally by the production strain for the desired polypeptide. However, in specific embodiments, the undesired side activity is derived from a fusion partner for the desired polypeptide.

In accordance with the invention, the medium as defined above is subjected to a pH In the range of 1.0 to 1.8 such as in the range of 1.5 to 1.8 or in the range of 1.7 to 1.8. In useful embodiments, the treatment is at a pH of 1.8.

The low pH treatment according to the invention is made for a period of time that is required to inactivate the side activity/activities to a desired level and that does not inactivate the biological activity of the desired polypeptide unacceptably. Typically, however, the required treatment period is within the range of 0.1 minutes to 48 hours such as a range of 1 minute to 36 hours including the range of 10 minutes to 24 hours. Those of skill in the art will be able to readily determine, for a particular polypeptide preparation, the appropriate treatment period using standard methods for assaying enzymatic activities and biological activity of the desired polypeptide.

The preparation being treated is a preparation containing aspartic protease activity, wherein the aspartic protease is chymosin. As mentioned above, the class of aspartic proteases includes industrially important milk clotting enzymes that are widely used in cheese manufacturing. Accordingly, media which are treated in accordance with the invention include media derived from the cultivation of a microorganism that during the cultivation produces an aspartic protease and at least one undesired enzymatic side activity such as a medium that is derived from the cultivation of a microorganism that naturally produces the aspartic protease or a medium that is derived from the cultivation of a recombinant microorganism that has an inserted gene expressing the aspartic protease.

Non-recombinant and recombinant microorganisms that are useful in the production of aspartic proteases include bacterial species and yeast species such as those mentioned above.

Species of filamentous fungi are also widely used for the production of milk clotting aspartic proteases and suitable filamentous fungi for that purpose include species of *Aspergil lus,* e.g. *Aspergillus oryzae, Aspergillus nidulans* or *Aspergillus niger* including *Aspergillus niger* var. *awamori.* Additionally, strains of a *Fusarium* species, e.g. *Fusarium oxysporum* or of a *Rhizomucor* species such as *Rhizomucor miehei* or a *Trichoderma* species including *Trichoderma reseei* and strains of *Cryphonectria* species including *Cryphonectria parasitica* can be used to produce milk clotting enzymes. Accordingly, the method of the present invention is used for inactivating side activities in media derived from cultivation of such fungal species.

In particular embodiments, the desired polypeptide in the preparation obtained herein is expressed a fusion protein having, in addition to the aspartic protease activity, at least one undesired enzymatic side activity such as an amylase or glucoamylase activity.

In accordance with the invention, the desired polypeptide in the preparation that is obtained is chymosin.

The aspartic protease can be derived from any mammal species such as a ruminant species including a bovine species, an ovine species, a caprine species, a deer species, a buffalo species, an antelope species and a giraffe species, a *Camelidae* species including *Camelus dromedarius,* a porcine species, an *Equidae* species and a primate species. As it is mentioned above, a commonly used milk clotting enzyme preparation is based on extracts of stomach tissues of mammals naturally producing a milk clotting aspartic protease. Accordingly, the method of the invention is applicable to such preparations, including preparations of aspartic proteases derived from a naturally produced aspartic protease by the addition or deletion of one or more amino acids or substituting one or more amino acids herein.

It is a further objective of the present invention to provide a milk clotting composition consisting of a preparation comprising an aspartic protease provided by the method of invention and which essentially does not have undesired enzymatic side activities including such activities selected from glucoamylase activity, lactase activity, starch degrading enzyme activity, protease activity, peptidase activity, phosphatase activity, lipase activity, cellulase activity and hemicellulase activity. Such compositions may, in addition to the active component contain one or more additives as conventionally used in the manufacturing of preparations of milk clotting enzymes (i.e. coagulants or rennets) derived from animal or microbial sources.

The invention will now be further explained in the following, non-limiting examples and the drawings where:
Fig. 1 illustrates the inactivation of glucoamylase activity in a fermentation medium for *Aspergillus nigervar. awamori* expressing bovine chymosin at different pH values, and

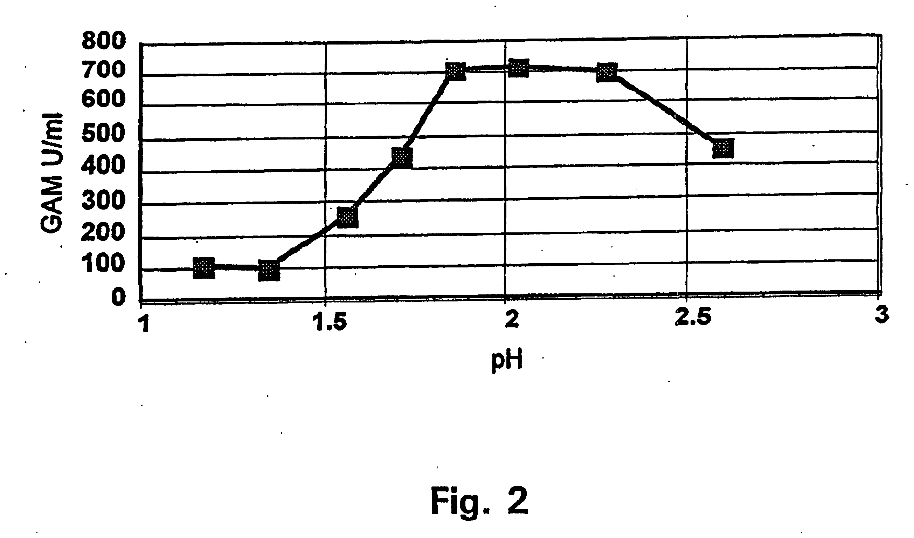

Fig. 2 shows the inactivation of Sigma glucoamylase at different pH values.

### EXAMPLE 1

### Inactivation of glucoamylase in a recombinant chymosin preparation

In this study, a filtrate derived from the cultivation of a recombinant strain of *Aspergillus niger* var. *awamori* expressing a prochymosin-glucoamylase fusion protein was used as test material. The filtrate was obtained from an industrial fermentation process by acidifying the cultivation medium after completion of the fermentation to inactivate the fungal biomass followed by separating the biomass by filtration.

The crude filtrate had a pH of about 2.2, contained 3113 U/ml of glucoamylase activity and had a conductivity of 19 mS (1 GAM unit is defined as the amount of activity that hydrolyses starch by generating 1 µg glucose per min under the below standard conditions). The milk clotting activity of the filtrate was 98.5 International Milk Clotting Units (IMCUs) per ml. Prior to testing, the filtrate was diluted 5 times with distilled water. As a control, a commercial preparation of pure glucoamylase (GAM) derived from *Aspergillus niger* (Sigma No. A3514) was included as a solution containing 0.05 mg/ml distilled water.

Eacn of the above test solutions were adjusted to 8 different pH values within the range of about 1.1 to 2.7 (2.76, 2.36, 2.03, 1.80, 1.61, 1.48, 1.30 and 1.15, respectively) and incubated at room temperature for about 20 hours. Following this incubation, the GAM activity was measured using a solution of starch (e.g. Merck Art 1257) prepared by dissolving 1 g in 100 ml 0.15 M acetate, pH 4.5 as substrate. For the assay, 500 µl of substrate solution, preheated in a water bath at 40°C, was mixed with 500 µl of enzyme solution to be tested and the mixture incubated for 20 minutes at 40°C. The reaction was stopped by the addition of 500 µl 0.3 M Tris. The amount of glucose formed was determined by mixing 200 µl of each of the reaction mixtures and 200 µl of a glucose standard containing 91 µg glucose/ml with 5.0 ml GOD-Perid reagent (Boehringer Mannheim 124028) reconstituted as recommended by the manufacturer. These mixtures were incubated at room temperature for 40 minutes followed by measuring E₆₁₀ using a spectrophotometer.

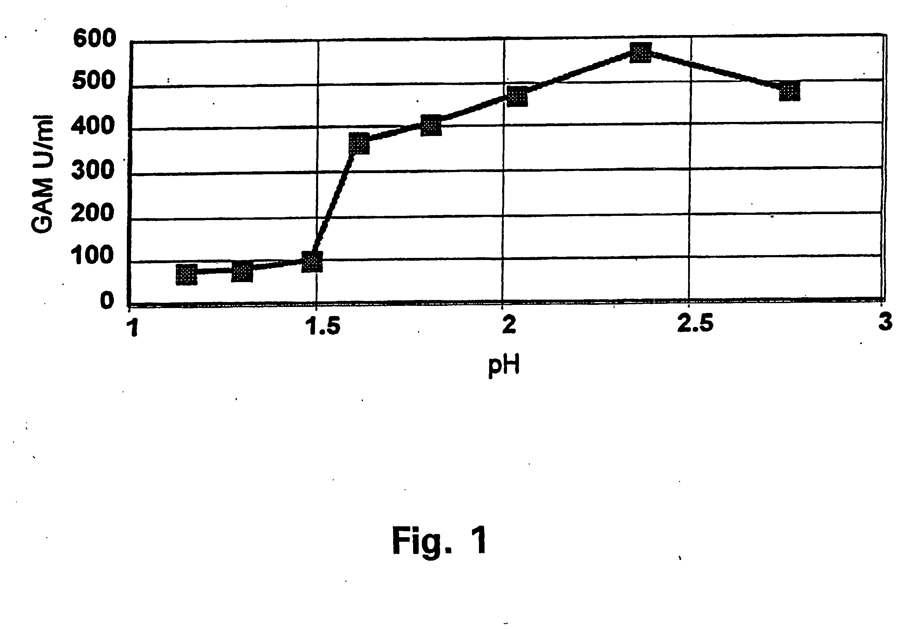

The results, which are summarised in Fig. 1 (filtrate) and 2 (Sigma GAM), demonstrate that it is possible to inactivate a substantial part of the GAM activity in the crude chymosin-containing preparation by subjecting the preparation to pH lower than 2.0.

### EXAMPLE 2

### Inactivation of enzymatic side activities in a filtrate of fermentation medium of a recombinant chymosin-producing Aspergillus niger var. awamori strain

The starting material for this experiment was a fresh fermentate of *Aspergillus niger* var. *awamori* transformed with a plasmid expressing a prochymosin-glucoamylase fusion protein. The fermentate contained a milk clotting activity of 157.5 IMCU/ml, pH 5.59.

Samples to be tested in the experiment were prepared as follows: To 600 ml of the crude fermentate 5.4 ml of 100% acetic acid (0.9%) was added and pH was adjusted to 2.5 by addition of concentrated sulphuric acid (5 ml). A 50 ml sample was withdrawn and pH further adjusted to 1.8, 1.7 and 1.6, respectively, and a 50 ml sample collected at each pH. As control, 50 ml of fermentate before pH adjustment was used.

Each of the above samples were centrifuged at 3.000 rpm for 10 minutes and subsequently filtered using a prefilter followed by filtration through a 0.45 µm filter. The samples were left to stand at 4°C for about 21 hours and the pH of each sample adjusted to 5.5-6.0 to stop further reaction.

Following the acid treatment, the respective samples were assayed for residual milk clotting activity according to International IDF Standard 157A:1997 and for the following enzymatic side activities: GAM, leucine aminopeptidase (LAP), amylase, cellulase, acid phosphatase and protease activity, respectively. GAM activity was assayed as in Example 1.

The LAP activity was assayed using Leu-β-naphtylamine as a substrate, which, in the presence of LAP activity, releases β-naphtylamine which is converted to a blue azo dye by first reacting it with NaNO₂ resulting in the generation of a diazo reagent which in a subsequent step is reacted with N-[1-naphtyl]-ethylenediamine to obtain the blue azo dye. In the assay, 125 µl of sample and 125 µl of LAP substrate is reacted for 1 hour at 37°C and the reaction is terminated by the addition of 125 µl of 2 N HCl. The colour reaction is measured spectrophotometrically and the LAP activity determined vs. a standard curve. One peptidase unit is defined as the amount of enzyme releasing 1 µmol of β-naphtylamine from Leu-β-naphtylamide per hour at 37°C and pH 7.1.

Amylase activity (1 unit defined as the amount of enzyme releasing 1 µmole of reducing groups calculated as maltose per minute under the below conditions) was assayed using soluble starch (Merck Art 1257) as the substrate at a concentration of 1% in 0.15 M sodium acetate, pH 4.8. Equal volumes of substrate and sample were mixed and incubated for 0 and 5 minutes, respectively at 25°C followed by addition of an equal volume of a colour reagent (1 g 3.5-dinitrosalicylic acid is suspended in 20 ml 2 N NaOH and 50 ml distilled water is added followed by the addition of 30.0 g PCNa tartrate and dilution to 100 ml) and boiling for 5 minutes followed by cooling in an ice bath. The enzyme activity was determined spectrophotometrically vs. a standard curve.

Cellulase activity was assayed using microcrystalline cellulose (Avicel FMC Corp.) as substrate using a GOD-Perid reagent (Boehringer Mannheim 124028) including a 91 µg/ml glucose standard. For the assay, 100 mg substrate, 2.0 ml 0.15 M sodium acetate buffer, pH 5.0 and 40 µl 5% Na azide was mixed and 0.5 ml of the sample added. The reaction mixture is incubated for 24 hours at 37°C followed by centrifugation. 200 µl of the resulting supernatant is mixed with 5.0 ml GOD-Perid reagents and the mixture kept at room temperature for 40 minutes and the colour development measured spectrophotometrically.

The assay for acid phosphatase is based on enzymatic hydrolysis of *p*-nitrophenyl phosphate. In the reaction, *p*-nitrophenol and inorganic phosphate are generated. When subjected to alkaline conditions, *p*-nitrophenol is converted to a yellow complex that can be measured at 400-420 nm. For the assay, a mixture of 0.5 ml substrate solution and 0.5 ml citrate buffer is prepared and 0.2 ml of sample is added followed by incubating the resulting mixture at 37°C for 30 minutes. The reaction is stopped by the addition of NaOH and the absorbance at 420 nm determined after 10 minutes.

General protease activity was measured using azo casein as the substrate. One unit of protease activity is defined as the amount of enzyme that provides a □ E₄₂₅ of 1.00 per minute at 30°C under defined conditions. For the assay, equal volumes of gel filtered sample at pH 5.2 and 5% azo casein at pH 6.7 is incubated in a 30°C water bath for exactly 30 minutes after which the reaction is stopped by adding 1.5 ml 5% TCA while mixing. The reaction tube is cooled in ice bath and centrifuged until a clear supernatant is obtained. One ml of the supernatant is mixed with 2 ml 4 NaOH and the extinction measured spectrophotometrially at 425 nm.

The contents of milk clotting activity in the samples of fermentate subjected to low pH for about 21 hours are summarised in the below table 2.1:

**Table 2.1. Milk clotting activity in samples of chymosin-producing Aspergillus niger var. awamori fermentate after 21 hours of low pH treatment**

| Sample | Milk clotting activity, IMCU/ml | Residual milk clotting activity, % |
|---|---|---|
| Filtrate of fermentate, pH 5.6 | 158 | 100 |
| Filtrate treated at pH 1.8 | 136 | 86.1 |
| Filtrate treated at pH 1.7 | 138 | 87.3 |
| Filtrate treated at pH 1.6 | 137 | 86.7 |

As it appears from the above table, more than 85% of the milk clotting activity of chymosin was retained even at pH 1.6.

In the following tables, the results for residual enzymatic side activities are summarised:

**Table 2.2. GAM activity in samples of chymosin-producing Aspergillus niger var. awamori fermentate after 21 hours of low pH treatment**

| Sample | GAM activity, U/ml | Residual GAM activity, % |
|---|---|---|
| Filtrate of fermentate, pH 5.6 | 30,619 | 100 |
| Filtrate treated at pH 1.8 | 4,020 | 13.1 |
| Filtrate treated at pH 1.7 | 234 | 0.8 |
| Filtrate treated at pH 1.6 | 0 | 0 |

**Table 2.3. Peptidase activity in samples of chymosin-producing Aspergillus niger var. awamori fermentate after 21 hours of low pH treatment**

| Sample | Peptidase activity, U/ml | Residual peptidase activity, % |
|---|---|---|
| Filtrate of fermentate, pH 5.6 | 209 | 100 |
| Filtrate treated at pH 1.8 | 0.3 | 0.1 |
| Filtrate treated at pH 1.7 | 0 | 0 |
| Filtrate treated at pH 1.6 | 0 | 0 |

**Table 2.4. Amylase activity in samples of chymosin-producing Aspergillus niger var. awamori fermentate after 21 hours of low pH treatment**

| Sample | Amylase activity, U/ml | Residual amylase activity, % |
|---|---|---|
| Filtrate of fermentate, pH 5.6 | 34 | 100 |
| Filtrate treated at pH 1.8 | 0.11 | 0.05 |
| Filtrate treated at pH 1.7 | 0.04 | 0.01 |
| Filtrate treated at pH 1.6 | 0.02 | 0 |

**Table 2.5. Cellulase activity in samples of chymosin-producing Aspergillus niger var. awamori fermentate after 21 hours of low pH treatment**

| Sample | Peptidase activity, U/ml | Residual peptidase activity, % |
|---|---|---|
| Filtrate of fermentate, pH 5.6 | 170 | 100 |
| Filtrate treated at pH 1.8 | 17.6 | 10.3 |
| Filtrate treated at pH 1.7 | 15.0 | 8.8 |
| Filtrate treated at pH 1.6 | 28.7 | 16.9 |

**Table 2.6. Phosphatase activity in samples of chymosin-producing Aspergillus niger var. awamori fermentate after 21 hours of low pH treatment**

| Sample | Phosphatase activity, U/ml | Residual phosphatase activity, % |
|---|---|---|
| Filtrate of fermentate, pH 5.6 | 706 | 100 |
| Filtrate treated at pH 1.8 | 397 | 56.2 |
| Filtrate treated at pH 1.7 | 401 | 56.8 |
| Filtrate treated at pH 1.6 | 372 | 52.7 |

**Table 2.7. Protease activity in samples of chymosin-groducing Aspergillus niger var. awamori fermentate after 21 hours of low pH treatment**

| Sample | Protease activity, U/ml | Residual protease activity, % |
|---|---|---|
| Filtrate of fermentate, pH 5.6 | 2 | 100 |
| Filtrate treated at pH 1.8 | 1 | 50 |
| Filtrate treated at pH 1.7 | 0 | - |
| Filtrate treated at pH 1.6 | 1 | 50 |

It appears from the above tables 2.2 to 2.7 that, with the exception of acid phosphatase activity, all the side activities being assayed were reduced by 50% or more by subjecting the fermentate to 21 hours of treatment at pH values below 2.0. At the same time, more than 85% of the milk clotting activity of chymosin was retained during that treatment.

### EXAMPLE 3

### Inactivation of enzymatic side activities in final ready-to-use rennet products by low pH treatment

In this experiment, samples of the following commercial microbial and animal rennet products were subjected to pH 1.7 for 2.5 hours and the GAM activity and the overall starch degrading activity was measured before and after that treatment.

The tested products were: Hannilase^{™} 195, a microbial coagulant produced by *Rhizomucor miehei,* Hannilase^{™} 2100, also a microbial rennet produced by *Rhizomucor miehei,* CHY-MAX^{™}, a bovine chymosin produced by *Aspergillus niger* var. *awamori,* Modilase^{™} 195, an oxidised, thermolabile coagulant derived from *Rhizomucor miehei* and Thermolase^{™}, a microbial coagulant produced by *Cryphonectria parasitica.*

The above products were tested for GAM activity using the assay described in Example 1 and for total starch degrading activity using an agar diffusion test. The results of this assay was indicated as +++, ++, + or (+), where (+) indicates trace activity only observed after extended incubation.

The results of this experiment are summarised in the below tables:

**Table 3.1. GAM activity in samples of commercial rennet products before and after low pH treatment**

| Rennet product | GAM activity, U/ml before low pH treatment | GAM activity, U/ml after low pH treatment | Residual GAM activity, % | |
|---|---|---|---|---|
| Hannilase^{™} 195 | 212 | 1.4 | 0.7 | |
| Hannilase^{™} 2100 | 1254 | 2.1 | 0.2 | 1) |
| CHY-MAX^{™} | 14 | 1.7 | 12.1 | |
| Modilase^{™} | 327 | 1.4 | 0.4 | 1) |
| Thermolase | 101 | 46 | 45.5 | 1) |

**Table 3.2. General starch degrading activity in samples of commercial rennet products before and after low pH treatment**

| Rennet product | Amylase activity before low pH treatment | Amylase activity after low pH treatment | Residual amylase activity, % | |
|---|---|---|---|---|
| Hannilase^{™} 195 | +++ | (+) | 0 | 1) |
| Hannilase^{™} 2100 | +++ | (+) | 0 | 1) |
| CHY-MAX^{™} | not detectable | not detectable | 0 | |
| Modilase^{™} | +++ | (+) | 0 | 1) |
| Thermolase | +++ | (+) | 0 | 1) |

| | | | | |
|---|---|---|---|---|
| 1) Reference examples | | | | |

It can be concluded from the above results that subjecting commercial rennet products of microbial and animal origin to a treatment at pH less than 2.0 can reduce the level of GAM activity significantly and in any case by more than 50%, and the treatment inactivates the total starch degrading activity to levels which are close to being undetectable by the used diffusion assay.

## Claims

1. A method of providing a polypeptide preparation having a reduced content of undesired enzymatic side activities, the method comprising the steps of:
(i) providing a medium having a pH of 2.0 or higher which is derived from the cultivation of an organism that is selected from the group consisting of a bacterial species, a yeast species and a species of filamentous fungi and that comprises at least one desired polypeptide which has aspartic protease activity, wherein the aspartic protease is chymosin, and in addition hereto at least one undesired enzymatic side activity, and
(ii) subjecting said medium to a pH in the range of 1.0 to 1.8 for a period of time that is sufficient to at least partially inactivate the at least one enzymatic side activity.

2. A method according to claim 1 wherein at least 75% of the activity of the at least one desired polypeptide is retained after subjecting the medium having a pH of 2.0 or more to a pH in the range of 1.0 to 1.8.

3. A method according to claim 2 wherein at least 85% of the activity of the at least one desired polypeptide is retained.

4. A method according to any of claims 1-3 wherein at least 50% of the activity of the at least one undesired enzymatic activity is inactivated.

5. A method according to claim 4 wherein at least 90% of the activity of the at least one undesired enzymatic activity is inactivated.

6. A method according to any of claims 1-5 wherein the medium having a pH of 2.0 or higher is a medium derived from the cultivation of an organism that during its cultivation produces the at least one desired polypeptide and the at least one undesired enzymatic side activity.

7. A method according to any of claims 1-6 wherein the at least one enzymatic side activity is selected from the group consisting of glucoamylase activity, starch degrading enzyme activity, protease activity, peptidase activity, phosphatase activity, lipase activity, cellulase activity, lactase activity and hemicellulase activity.

8. A method according to claim 1 wherein the bacterial species is selected from the group consisting of a gram negative bacterial species including E. *coli* and a gram positive species including a Bacillus species.

9. A method according to claim 1 wherein the yeast species is selected from the group consisting of Saccharomyces cerevisiae, a methylotrophic yeast species including *Pichia pastoris* and a *Klyuveromyces* species including *Klyuveromyces lactis.*

10. A method according to claim 1 wherein the species of filamentous fungi is selected from the group consisting of an *Aspergillus* species, a *Cryphonectria* species, a *Fusarium* species, a *Rhizomucor* species and a *Trichoderma* species.

11. A method according to any of claims 1-10 wherein the pH is in the range of 1.5 to 1.8.

12. A method according to claim 11 wherein the pH is in the range of 1.7 to 1.8.

13. A method according to claim 12 wherein the pH is 1.8.

14. A method according to any of claims 11-13 wherein the pH in the range of 1.0 to 1.8 is provided by adding an inorganic or an organic acid.

15. A method according to any of claims 1-14 wherein the medium having a pH of 2.0 or higher is subjected to a pH in the range of 1.0 to 1.8 for a period of time that is in the range of 0.1 minutes to 48 hours.

16. A method according to any preceding claim wherein the medium having a pH of 2.0 or higher is a medium derived from the cultivation of a microorganism that during the cultivation produces the aspartic protease and the at least one undesired enzymatic side activity.

17. A method according to claim 16 wherein the medium is derived from the cultivation of a microorganism that naturally produces the aspartic protease or from the cultivation of a recombinant microorganism that has an inserted gene expressing the aspartic protease.

18. A method according to claim 17 wherein the microorganism is selected from the group consisting of a bacterial species, a yeast species and a species of filamentous fungi.

19. A method according to claim 18 wherein the aspartic protease is expressed as a fusion protein having, in addition to the aspartic protease activity, at least one undesired enzymatic side activity.

20. A method according to claim 19 wherein the at least one enzymatic side activity is starch degrading enzyme activity including an activity selected from the group consisting of amylase activity and glucoamylase activity.

21. A method according to any of claims 16-20 wherein the microorganism is one that naturally produces at least one enzymatic side activity.

22. A method according to claim 21 wherein the at least one enzymatic side activity is selected from the group consisting of glucoamylase activity, lactase activity, starch degrading enzyme activity, protease activity, peptidase activity, phosphatase activity, lipase activity, cellulase activity and hemicellulase activity.

23. A method according to claim 1 wherein the chymosin is derived from a mammalian species selected from the group consisting of a ruminant species, a *Camelidae* species including *Camelus dromedarius,* a porcine species, an *Equidae* species and a primate species.

24. A method according to claim 23 wherein the ruminant species is selected from the group consisting of a bovine species, an ovine species, a caprine species, a deer species, a buffalo species, an antelope species and a giraffe species.

25. A method according to any of claims 23-24 wherein the chymosin is a protease naturally produced in a mammalian species.

26. A method according to claim 1 wherein the aspartic protease is derived from a naturally produced aspartic protease by the addition or deletion of one or more amino acids or substitution of one or more amino acids herein.

## Patentansprüche

1. Verfahren zur Herstellung eines Polypeptidpräparats mit einem verringerten Gehalt an unerwünschten enzymatischen Nebenaktivitäten, wobei das Verfahren die folgenden Schritte umfasst:
(i) Herstellen eines Mediums mit einem pH-Wert von 2,0 oder höher, das aus der Kultivierung eines Organismus gewonnen wird, der aus der Gruppe ausgewählt ist, die aus einer Bakterienart, einer Hefeart und einer fadenförmigen Pilzart besteht, und das mindestens ein erwünschtes Polypeptid, das aspartische Proteaseaktivität aufweist, wobei die aspartische Protease Chymosin ist, und zusätzlich dazu mindestens eine unerwünschte enzymatische Nebenaktivität umfasst, und
(ii) Aussetzen dieses Mediums einem pH-Wert im Bereich von 1,0 bis 1,8 über eine Zeitspanne, die ausreicht, um die mindestens eine enzymatische Nebenaktivität zumindest teilweise zu deaktivieren.

2. Verfahren nach Anspruch 1, bei dem mindestens 75% der Aktivität des mindestens einen erwünschten Polypeptids beibehalten werden, nachdem das Medium mit einem pH-Wert von 2,0 oder mehr einem pH-Wert im Bereich von 1,0 bis 1,8 ausgesetzt wurde.

3. Verfahren nach Anspruch 2, bei dem mindestens 85% der Aktivität des mindestens einen erwünschten Polypeptids beibehalten werden.

4. Verfahren nach einem beliebigen der Ansprüche 1-3, bei dem mindestens 50% der Aktivität der mindestens einen unerwünschten enzymatischen Aktivität deaktiviert werden.

5. Verfahren nach Anspruch 4, bei dem mindestens 90% der Aktivität der mindestens einen unerwünschten enzymatischen Aktivität deaktiviert werden.

6. Verfahren nach einem beliebigen der Ansprüche 1-5, bei dem das Medium mit einem pH-Wert von 2,0 oder höher ein Medium ist, das aus der Kultivierung eines Organismus gewonnen wird, der während seiner Kultivierung das mindestens eine erwünschte Polypeptid und die mindestens eine unerwünschte enzymatische Nebenaktivität produziert.

7. Verfahren nach einem beliebigen der Ansprüche 1-6, bei dem die mindestens eine enzymatische Nebenaktivität aus folgender Gruppe ausgewählt ist: Glucoamylaseaktivität, Aktivität stärkeabbauender Enzyme, Proteaseaktivität, Peptidaseaktivität, Phosphataseaktivität, Lipaseaktivität, Cellulaseaktivität, Lactaseaktivität und Hemicellulaseaktivität.

8. Verfahren nach Anspruch 1, bei dem die Bakterienart aus folgender Gruppe ausgewählt ist: eine gramnegative Bakterienart einschließlich *E. coli* und eine grampositive Art einschließlich einer Bazillusart.

9. Verfahren nach Anspruch 1, bei dem die Hefeart aus folgender Gruppe ausgewählt ist: *Saccharomyces cerevisiae,* eine methylotrophische Hefeart einschließlich *Pichia pastoris* und eine *Klyuveromyces-*Art einschließlich *Klyuveromyces lactis.*

10. Verfahren nach Anspruch 1, bei dem die fadenförmige Pilzart aus folgender Gruppe ausgewählt ist: eine *Aspergillus-Art,* eine *Cryphonectria-Art,* eine *Fusarium-*Art, eine *Rhizomucor-Art* und eine *Trichoderma-Art.*

11. Verfahren nach einem beliebigen der Ansprüche 1-10, bei dem der pH-Wert im Bereich von 1,5 bis 1,8 liegt.

12. Verfahren nach Anspruch 11, bei dem der pH-Wert im Bereich von 1,7 bis 1,8 liegt.

13. Verfahren nach Anspruch 12, bei dem der pH-Wert 1,8 ist.

14. Verfahren nach einem beliebigen der Ansprüche 11-13, bei dem der pH-Wert im Bereich von 1,0 bis 1,8 liegt, indem man eine anorganische oder organische Säure hinzufügt.

15. Verfahren nach einem beliebigen der Ansprüche 1-14, bei dem das Medium mit einem pH-Wert von 2,0 oder höher einem pH-Wert im Bereich von 1,0 bis 1,8 über eine Zeitspanne im Bereich von 0,1 Minuten bis 48 Stunden ausgesetzt wird.

16. Verfahren nach einem beliebigen der vorhergehenden Ansprüche, bei dem das Medium mit einem pH-Wert von 2,0 oder höher ein Medium ist, das aus der Kultivierung eines Mikroorganismus gewonnen wird, der während der Kultivierung die aspartische Protease und die mindestens eine unerwünschte enzymatische Nebenaktivität produziert.

17. Verfahren nach Anspruch 16, bei dem das Medium aus der Kultivierung eines Mikroorganismus gewonnen wird, der die aspartische Protease natürlich produziert, oder aus der Kultivierung eines rekombinanten Mikroorganismus, der ein eingefügtes Gen hat, das die aspartische Protease ausdrückt.

18. Verfahren nach Anspruch 17, bei dem der Mikroorganismus aus folgender Gruppe ausgewählt ist: eine Bakterienart, eine Hefeart und eine fadenförmige Pilzart.

19. Verfahren nach Anspruch 18, bei dem die aspartische Protease als ein Fusionsprotein ausgedrückt ist, mit, zusätzlich zu der aspartischen Proteaseaktivität, mindestens einer unerwünschten enzymatischen Nebenaktivität.

20. Verfahren nach Anspruch 19, bei dem die mindestens eine enzymatische Nebenaktivität Aktivität stärkeabbauender Enzyme einschließlich einer Aktivität ausgewählt aus der Gruppe bestehend aus Amylasenaktivität ist und Glucoamylaseaktivität ist.

21. Verfahren nach einem beliebigen der Ansprüche 16-20, bei dem der Mikroorganismus einer ist, der mindestens eine enzymatische Nebenaktivität natürlich produziert.

22. Verfahren nach Anspruch 21, bei dem die mindestens eine enzymatische Nebenaktivität aus folgender Gruppe ausgewählt ist: Glucoamylaseaktivität, Lactaseaktivität, Aktivität stärkeabbauender Enzyme, Proteaseaktivität, Peptidaseaktivität, Phosphataseaktivität, Lipaseaktivität, Cellulaseaktivität und Hemicellulaseaktivität.

23. Verfahren nach Anspruch 1, bei dem das Chymosin von einer Säugetierart gewonnen wird, die aus folgender Gruppe ausgewählt ist: einer Wiederkäuerart, einer *Camelidae*-Art einschließlich *Camelus dromedarius,* einer Schweine-Art, einer *Equidae-Art* und einer Primatenart.

24. Verfahren nach Anspruch 23, bei dem die Wiederkäuerart aus folgender Gruppe ausgewählt ist: eine Rinderart, eine Schafsart, eine Ziegen-Art, eine Hirschart, eine Büffelart, eine Antilopenart und eine Giraffenart.

25. Verfahren nach einem beliebigen der Ansprüche 23-24, bei dem das Chymosin eine in einer Säugetierart natürlich produzierte Protease ist.

26. Verfahren nach Anspruch 1, bei dem die aspartische Protease aus einer natürlich produzierten aspartischen Protease durch die Zugabe oder Deletion einer oder mehrerer Aminosäuren oder Austausch einer oder mehrerer Aminosäuren darin gewonnen wird.

## Revendications

1. Procédé de fourniture d'une préparation de polypeptides présentant un contenu réduit d'activités secondaires enzymatiques non désirées, le procédé comprenant les étapes consistant à :
(i) fournir un milieu présentant un pH de 2,0 ou supérieur qui est dérivé de la culture d'un organisme qui est sélectionné dans le groupe consistant en une espèce bactérienne, une espèce de levure et une espèce de champignon filamenteux et qui comprend au moins un polypeptide désiré qui présente une activité de protéase aspartique, dans lequel la protéase aspartique est la chymosine, et en plus au moins une activité secondaire enzymatique non désirée, et
(ii) soumettre ledit milieu à un pH dans la gamme allant de 1,0 à 1,8 pendant une période de temps qui est suffisante pour inactiver au moins partiellement la au moins une activité secondaire enzymatique.

2. Procédé selon la revendication 1 dans lequel au moins 75 % de l'activité du au moins un polypeptide désiré sont retenus après avoir soumis le milieu présentant un pH de 2,0 ou plus à un pH dans la gamme allant de 1,0 à 1,8.

3. Procédé selon la revendication 2 dans lequel au moins 85 % de l'activité du au moins un polypeptide désiré sont retenus.

4. Procédé selon l'une quelconque des revendications 1-3 dans lequel au moins 50 % de l'activité de la au moins une activité enzymatique non désirée sont inactivés.

5. Procédé selon la revendication 4 dans lequel au moins 90 % de l'activité de la au moins une activité enzymatique non désirée sont inactivés.

6. Procédé selon l'une quelconque des revendications 1-5 dans lequel le milieu présentant un pH de 2,0 ou supérieur est un milieu dérivé de la culture d'un organisme qui pendant sa culture produit le au moins un polypeptide désiré et la au moins une activité secondaire enzymatique non désirée.

7. Procédé selon l'une quelconque des revendications 1-6 dans lequel la au moins une activité secondaire enzymatique est sélectionnée dans le groupe consistant en une activité de glucoamylase, une activité d'enzyme de dégradation d'amidon, une activité de protéase, une activité de peptidase, une activité de phosphatase, une activité de lipase, une activité de cellulase, une activité de lactase et une activité d'hémicellulase.

8. Procédé selon la revendication 1 dans lequel l'espèce bactérienne est sélectionnée dans le groupe consistant en une espèce bactérienne à Gram négatif incluant *E. coli* et une espèce à Gram positif incluant une espèce de Bacillus.

9. Procédé selon la revendication 1 dans lequel l'espèce de levure est sélectionnée dans le groupe consistant en *Saccharomyces cerevisiae,* une espèce méthylotrophique de levure incluant *Pichia pastoris* et une espèce de *Klyuveromyces* incluant *Klyuveromyces lactis.*

10. Procédé selon la revendication 1 dans lequel l'espèce de champignon filamenteux est sélectionnée dans le groupe consistant en une espèce *d'Aspergillus,* une espèce de *Cryphonectria,* une espèce de *Fusarium,* une espèce de *Rhizomucor* et une espèce de *Trichoderma.*

11. Procédé selon l'une quelconque des revendications 1-10 dans lequel le pH est dans la gamme allant de 1,5 à 1,8.

12. Procédé selon la revendication 11 dans lequel le pH est dans la gamme allant de 1,7 à 1,8.

13. Procédé selon la revendication 12 dans lequel le pH est de 1,8.

14. Procédé selon l'une quelconque des revendications 11-13 dans lequel le pH dans la gamme allant de 1,0 à 1,8 est fourni en ajoutant un acide inorganique ou organique.

15. Procédé selon l'une quelconque des revendications 1-14 dans lequel le milieu présentant un pH de 2,0 ou supérieur est soumis à un pH dans la gamme allant de 1,0 à 1,8 pendant une période de temps qui est dans la gamme allant de 0,1 minute à 48 heures.

16. Procédé selon une quelconque revendication précédente dans lequel le milieu présentant un pH de 2,0 ou supérieur est un milieu dérivé de la culture d'un microorganisme qui pendant la culture produit la protéase aspartique et la au moins une activité secondaire enzymatique non désirée.

17. Procédé selon la revendication 16 dans lequel le milieu est dérivé de la culture d'un microorganisme qui produit naturellement la protéase aspartique ou de la culture d'un microorganisme recombinant qui présente un gène inséré exprimant la protéase aspartique.

18. Procédé selon la revendication 17 dans lequel le microorganisme est sélectionné dans le groupe consistant en une espèce bactérienne, une espèce de levure et une espèce de champignon filamenteux.

19. Procédé selon la revendication 18 dans lequel la protéase aspartique est exprimée sous la forme d'une protéine de fusion présentant, en plus de l'activité de protéase aspartique, au moins une activité secondaire enzymatique non désirée.

20. Procédé selon la revendication 19 dans lequel la au moins une activité secondaire enzymatique est une activité d'enzyme de dégradation d'amidon incluant une activité sélectionnée dans le groupe consistant en une activité d'amylase et une activité de glucoamylase.

21. Procédé selon l'une quelconque des revendications 16-20 dans lequel le microorganisme est un qui produit naturellement au moins une activité secondaire enzymatique.

22. Procédé selon la revendication 21 dans lequel la au moins une activité secondaire enzymatique est sélectionnée dans le groupe consistant en une activité de glucoamylase, une activité de lactase, une activité d'enzyme de dégradation d'amidon, une activité de protéase, une activité de peptidase, une activité de phosphatase, une activité de lipase, une activité de cellulase et une activité d'hémicellulase.

23. Procédé selon la revendication 1 dans lequel la chymosine est dérivée d'une espèce de mammifère sélectionnée dans le groupe consistant en une espèce de ruminant, une espèce de *Camelidae* incluant *Camelus dromedorius,* une espèce porcine, une espèce d'*Equidae* et une espèce de primate.

24. Procédé selon la revendication 23 dans lequel l'espèce de ruminant est sélectionnée dans le groupe consistant en une espèce bovine, une espèce ovine, une espèce de caprin, une espèce de cerf, une espèce de buffle, une espèce d'antilope et une espèce de girafe.

25. Procédé selon l'une quelconque des revendications 23-24 dans lequel la chymosine est une protéase produite naturellement dans une espèce de mammifère.

26. Procédé selon la revendication 1 dans lequel la protéase aspartique est dérivée d'une protéase aspartique naturellement produite par l'addition ou la délétion d'un ou plusieurs acides aminés ou la substitution d'un ou plusieurs acides aminés.
